# EUROPEAN PATENT APPLICATION

(11) **EP 3 827 827 A1**
(43) Date of publication of application: **02.06.2021**
(21) Application number: 19840735.5
(22) Date of filing: 25.07.2019
(51) Int. Cl.: A61K 31/427, A61P 19/02, A23L 33/10

(54) **PHARMACEUTICAL COMPOSITION FOR PREVENTING, ALLEVIATING OR TREATING OSTEOARTHRITIS, CONTAINING RHODANINE DERIVATIVE AS ACTIVE INGREDIENT**

(30) Priority: 25.07.2018 KR 20180086505
(71) Applicant: Avixgen Inc., Seoul 06591 (KR)
(72) Inventor: KIM, Min Jung, Seoul 08785 (KR)
(74) Representative: Nony
(86) International application number: PCT/KR2019/009213
(87) International publication number: WO 2020/022789

(57) **Abstract**

The present disclosure relates to the use of a rhodanine derivative in the prevention, amelioration or treatment of osteoarthritis. The rhodanine derivative according to the present disclosure has excellent effects of rescuing the loss of the cartilage matrix and alleviating synovitis, and thus may be effectively used for the prevention, amelioration or treatment of osteoarthritis.

## Description

### Technical Field

The present disclosure relates to a pharmaceutical composition for preventing, ameliorating or treating osteoarthritis, the pharmaceutical composition containing a rhodanine derivative as an active ingredient.

### Background Art

In the modern society, patients with degenerative diseases such as dementia, arthritis, and arteriosclerosis are increasing with the aging of the population, and the limitation of social activity of the elderly population due to these degenerative diseases causes economic and mental difficulties for the patients themselves, but also for their families, leading to social problems. Among them, osteoarthritis, a senile disease which can be commonly seen in clinical practice due to the population aging trend, is a chronic degenerative disease which is caused by progressive damage to articular cartilage due to degeneration of the extracellular matrix constituting the articular cartilage and involves inflammation and pain.

The morbidity of osteoarthritis increases with age, and it has been reported that cartilage abnormalities appear in more than 60% of people aged 60 years or older (Bjelle (1982) Scand. J. Rheumatol. Suppl., 43:35-48). It is known that, as Korea has recently entered an aging society, about 80% of people over the age of 55 and almost the majority of people over the age of 75 have osteoarthritis diseases.

Currently, an effective method for treating osteoarthritis is unknown, and treatment is provided to reduce pain caused by osteoarthritis and to maintain normal joints by preventing destruction and deformation of joints. For example, when the pain of osteoarthritis is relatively mild, a drug such as acetaminophen is used, but if the pain persists or is severe or accompanied by inflammation even when the acetaminophen is administered, nonsteroidal anti-inflammatory drugs (NSAIDs) are mainly prescribed. However, the use of the nonsteroidal anti-inflammatory drugs is limited due to a high risk of serious gastrointestinal complications. Meanwhile, the use of glucosamine or chondroitin for control of pain in osteoarthritis patients has been recommended by the European League against Rheumatism, but there is still a lot of controversy about the safety thereof.

Accordingly, the present inventors have conducted studies to discover an effective therapeutic agent for the treatment of osteoarthritis, thereby completing the present disclosure.

### DISCLOSURE

### Technical Problem

One aspect is intended to provide a composition for preventing, ameliorating or treating osteoarthritis, the composition containing a rhodanine derivative compound or a pharmaceutically acceptable salt thereof as an active ingredient.

Another aspect is intended to provide a method of preventing, ameliorating or treating osteoarthritis using a rhodanine derivative compound.

### Technical Solution

One aspect provides a pharmaceutical composition for preventing or treating osteoarthritis, the pharmaceutical composition containing a compound represented by the following Formula 1 or a pharmaceutically acceptable salt thereof:

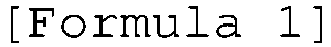

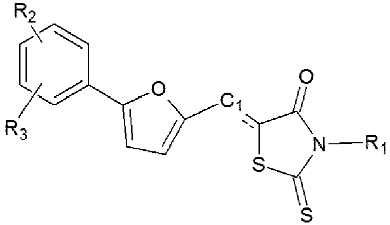

wherein R₁ is H, C₁₋₅ alkyl, C₁₋₅ alkoxy, -C₁₋₅ alkyl-carboxylic acid, -C₁₋₅ alkyl-C (=O) -C₁₋₃ alkyl, -C(=O) -C₁₋₅ alkyl, -C₁₋₂ alkyl-SO₂-C₁₋₂ alkyl, -C₁₋₂ alkyl-tetrahydrofuran, -phenyl, -C₁₋₂ alkyl-phenyl, -cyclohexyl, -dioxotetrahydrothiophene,-pyrazole, -C₁₋₃ alkyl-furan, or -CH (CO₂H) -CH₂-CO₂H, wherein the phenyl is unsubstituted or substituted with at least one substituent selected from the group consisting of C₁₋₃ alkyl, an amino group substituted with one or more C₁₋₂ alkyl groups, a halogen, -CF₃, and -NO₂; R₂ is H, a halogen, C₁₋₃ alkyl, or -NO₂, or is linked to R₃ to form a ring; R₃ is either any one of H, a halogen, C₁₋₃ alkyl, C₁₋₃ alkoxy, -C₁₋₅ alkyl-carboxylic acid, -C(=O)-O-R₄, -NO₂, and morpholine, or -C(=O)-NH-C(=O)-, -CH₂-O-C(=O)- or -C=C-C=C-, which is in the ortho position to R₂ and is linked to R₂ to form a ring; R₄ is a C₁₋₄ alkyl group; and C₁ is a sp³ hybrid carbon or a sp² hybrid carbon.

The alkyl group may be straight or branched.

Osteoarthritis is a disease accompanied by progressive destruction of joint cartilage and secondary symptoms associated therewith. Specifically, osteoarthritis may damage the bones and ligaments constituting the joints due to progressive damage to or degenerative changes in cartilage protecting the joint and may cause inflammation and pain. The compound represented by Formula 1 may effectively ameliorate symptoms of osteoarthritis such as loss of cartilage matrix and synovitis, and thus may be effectively used for the prevention or treatment of osteoarthritis.

In one embodiment, R₁ may have carboxylic acid and/or a ketone group.

In one embodiment, R₁ may be -CH₂CH₂C(=O)CH₃.

In one embodiment, R₂ may be in the para position to the furan.

According to one embodiment, the compound represented by Formula 1 may be a compound selected from the group consisting of
1) 3-{5-[5-(4-chlorophenyl)-furan-2-ylmethylene]-4-oxo-2-thioxo-thiazolidin-3-yl}-propionic acid,
2) 4-(5-((5-(4-(isopropoxycarbonyl)phenyl)furan-2-yl)methylene)-4-oxo-2-thioxothiazolidin-3-yl)butanoic acid,
3) 3-(5-((5-(4-chlorophenyl)furan-2-yl)methyl)-2,4-dioxothiazolidin-3-yl)propionic acid,
4) 3-(2,4-dimethylphenyl)-5-[5-(3-nitrophenyl)-furan-2-ylmethylene]-2-thioxothiazolidine-4-one,
5) 5-[5-(4-morpholine-4-yl-3-nitrophenyl)-furan-2-ylmethylene]-2-thioxothiazolidine-4-one,
6) 5-[5-(4-oxo-2-thioxothiazolidine-5-ylidenemethyl)-furan-2-yl]-isoindole-1,3-dione,
7) 3-(2-methanesulfonylethyl)-5-[5-(3-nitrophenyl)-furan-2-ylmethylene]-2-thioxothiazolidine-4-one,
8) 5-[5-(4-nitrophenyl)-furan-2-ylmethylene]-3-(tetrahydrofuran-2-ylmethyl)-2-thioxothiazolidine-4-one,
9) 3-(4-diethylaminophenyl)-5-[5-(4-nitrophenyl)-furan-2-ylmethylene]-2-thioxothiazolidine-4-one,
10) 5-[5-(2,4-dichlorophenyl)-furan-2-ylmethylene]-3-(3-fluorophenyl)-2-thioxothiazolidine-4-one,
11) 3-phenethyl-5-(5-phenyl-furan-2-ylmethylene)-2-thioxothiazolidine-4-one,
12) 5-[5-(3-chloro-4-methylphenyl)-furan-2-ylmethylene]-3-ethyl-2-thioxothiazolidine-4-one,
13) 5-(naphthalen-1-yl)-furan-2-ylmethylene-3-(2-oxopropyl)-2-thioxothiazolidine-4-one,
14) 3-cyclohexyl-5-[5-(2-nitrophenyl)-furan-2-ylmethylene]-2-thioxothiazolidine-4-one,
15) 3-(3-fluorophenyl)-5-[5-(4-nitrophenyl)-furan-2-ylmethylene]-2-thioxothiazolidine-4-one,
16) 3-(1,1-dioxotetrahydrothiophen-3-yl)-5-[5-(2-nitrophenyl)-furan-2-ylmethylene]-2-thioxothiazolidine-4-one,
17) 5-[5-(2-nitrophenyl)-furan-2-ylmethylene]-3-(4-oxopentyl)-2-thioxothiazolidine-4-one,
18) 5-[5-(2-bromo-5-nitrophenyl)furan-2-ylmethylene]-3-(4-iodophenyl)-2-thioxothiazolidine-4-one,
19) 5-[5-(2-chloro-4-nitrophenyl)furan-2-ylmethylene]-2-thioxo-3-(3-trifluoromethylphenyl)-thiazolidine-4-one,
20) 3-benzyl-5-[5-(4-ethoxy-2-nitrophenyl)-furan-2-ylmethylene]-2-thioxothiazolidine-4-one,
21) {5-[5-(2-methyl-5-nitrophenyl)furan-2-ylmethylene]-4-oxo-2-thioxothiazolidine-3-yl}acetic acid,
22) {5-[5-(4-ethoxy-2-nitrophenyl)-furan-2-ylmethylene]-4-oxo-2-thioxothiazolidine-3-yl}-acetic acid,
23) {5-[5-(4-methoxy-2-nitrophenyl)-furan-2-ylmethylene]-4-oxo-2-thioxothiazolidine-3-yl}acetic acid,
24) 3-(4-nitrophenyl)-5-[5-(4-nitrophenyl)-furan-2-ylmethylene]-2-thioxothiazolidine-4-one,
25) 4-[5-(4-oxo-2-thioxothiazolidine-5-ylidenemethyl)-furan-2-yl]-benzoicisopropylester,
26) 5-[5-(2,5-dichlorophenyl)furan-2-ylmethylene]-3-(1,5-dimethyl-3-oxo-2-phenyl-2,3-dihydro-1H-pyrazole-4-yl)-2-thioxothiazolidine-4-one,
27) 5-[5-(1-oxo-1,3-dihydroisobenzofuran-5-yl)-furan-2-ylmethylene]-2-thioxothiazolidine-4-one,
28) 3-(furan-2-ylmethyl)-5-[5-(2-nitrophenyl)furan-2-ylmethylene]-2-thioxothiazolidine-4-one,
29) 5-[5-(2-nitrophenyl)-furan-2-ylmethylene]-3-phenethyl-2-thioxothiazolidine-4-one,
30) 3-(3-chlorophenyl)-5-[5-(2,3-dichlorophenyl)-furan-2-ylmethylene]-2-thioxothiazolidine-4-one,
31) 2-chloro-5-[5-(4-oxo-2-thioxothiazolidine-5-ylidenemethyl)-furan-2-yl]-benzoicpropylester,
32) 4-{5-[5-(3,4-dichlorophenyl)-furan-2-ylmethylene]-4-oxo-2-thioxothiazolidine-3-yl}-butanoic acid,
33) 2-{5-[5-(4-nitrophenyl)-furan-2-ylmethylene]-4-oxo-2-thioxothiazolidine-3-yl}-succinic acid,
34) 5-[5-(4-chlorophenyl)furan-2-ylmethylene]-3-(furan-2-ylmethyl)-2-thioxothiazolidine-4-one,
35) 5-[5-(2,4-dichlorophenyl)furan-2-ylmethylene]-2-thioxo-3-(3-trifluoromethylphenyl)thiazolidine-4-one,
36) 3-(furan-2-ylmethyl)-5-[5-(4-nitrophenyl)furan-2-ylmethylene]-2-thioxothiazolidine-4-one,
37) 3-{5-[5-(3-nitrophenyl)furan-2-ylmethylene]-4-oxo-2-thioxothiazolidine-3-yl}-propionic acid,
38) 3-{5-[5-(4-chlorophenyl)furan-2-ylmethylene]-4-oxo-2-thioxothiazolidine-3-yl}-propionic acid,
39) 4-{5-[3-(2,4-dimethylphenyl)-4-oxo-2-thioxothiazolidin-5-ylidenemethyl]-furan-2-yl}benzoic acid,
40) {5-[5-(3-nitrophenyl)furan-2-ylmethylene]-4-oxo-2-thioxothiazolidin-3-yl}acetic acid, and
41) 5-[5-(4-bromophenyl)-furan-2-ylmethylene]-3-(3-oxobutyl)-2-thioxothiazolidine-4-one (hereinafter, these compounds may be referred to as compounds 1 to 41).

The formulae of compounds 1 to 41 are shown in Table 1 below.

**[Table 1]**

| Compound | Structure |
|---|---|
| 1 | 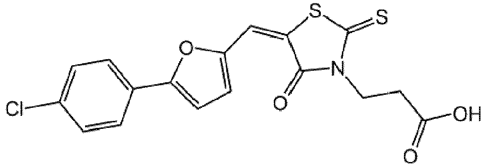 |
| 2 | 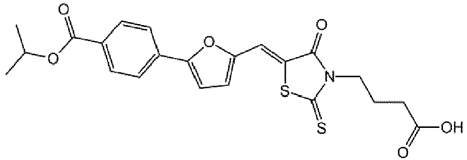 |
| 3 | 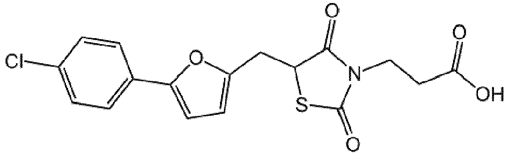 |
| 4 | 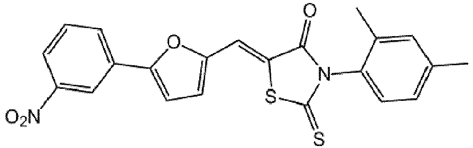 |
| 5 | 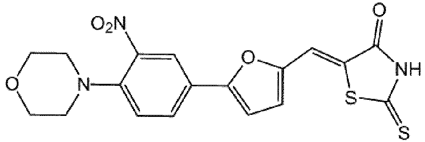 |
| 6 | 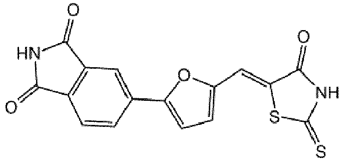 |
| 7 | 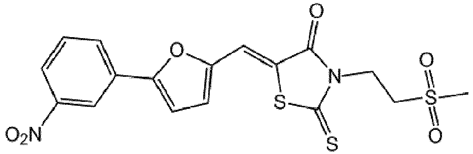 |
| 8 | 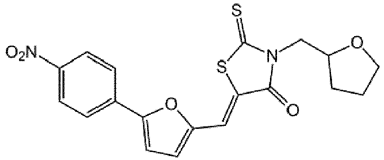 |
| 9 | 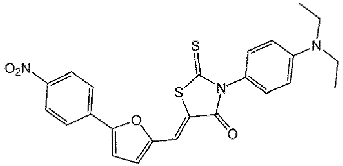 |
| 10 | 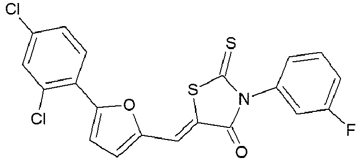 |
| 11 | 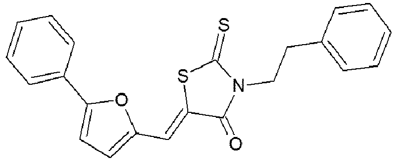 |
| 12 | 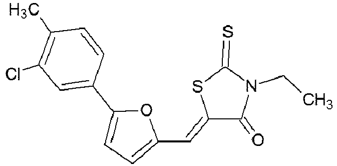 |
| 13 | 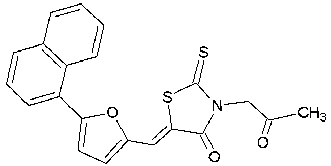 |
| 14 | 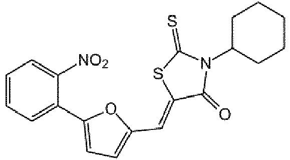 |
| 15 | 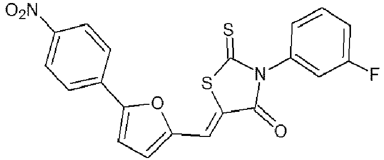 |
| 16 | 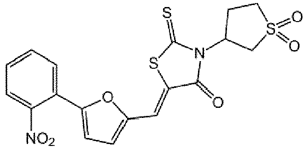 |
| 17 | 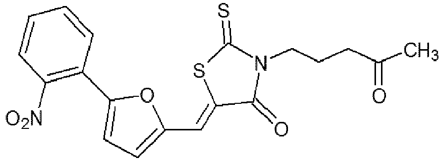 |
| 18 | 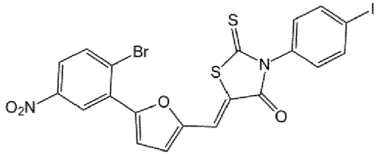 |
| 19 | 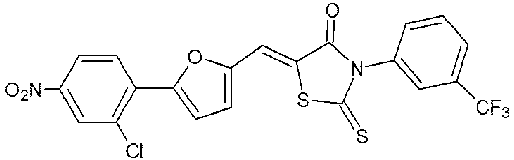 |
| 20 | 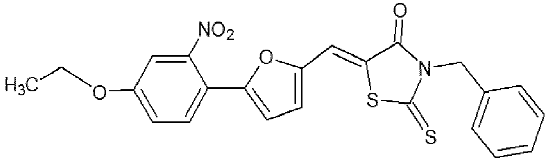 |
| 21 | 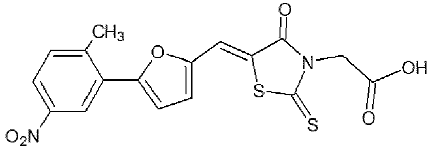 |
| 22 | 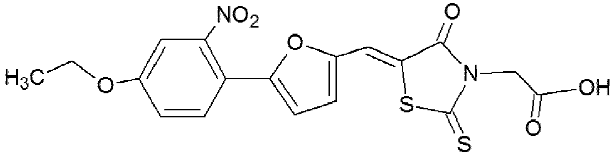 |
| 23 | 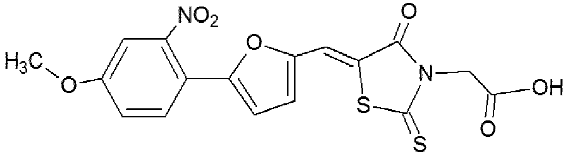 |
| 24 | 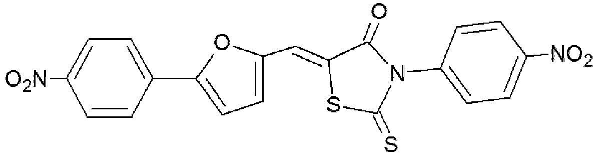 |
| 25 | 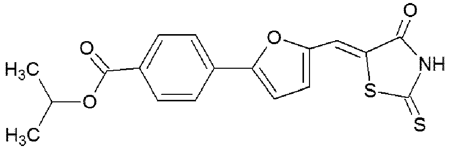 |
| 26 | 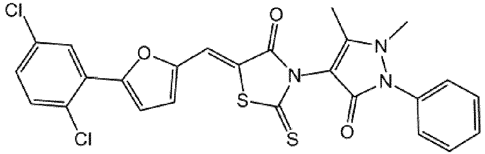 |
| 27 | 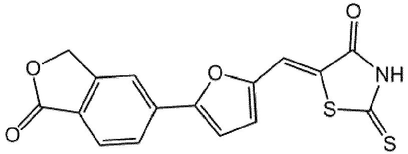 |
| 28 | 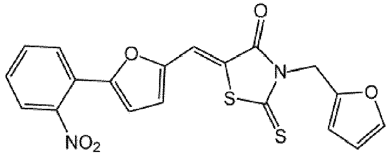 |
| 29 | 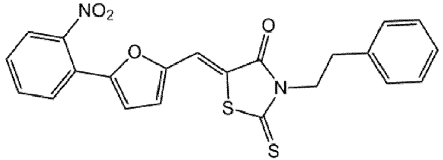 |
| 30 | 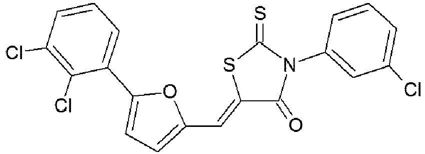 |
| 31 | 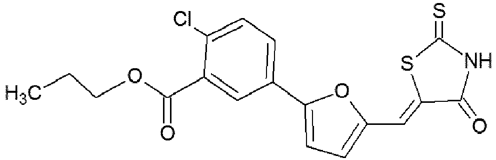 |
| 32 | 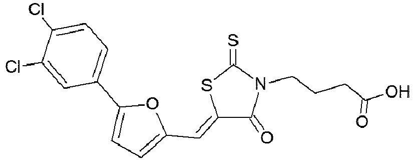 |
| 33 | 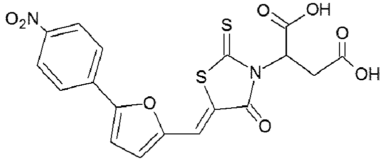 |
| 34 | 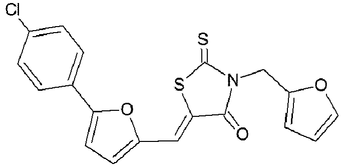 |
| 35 | 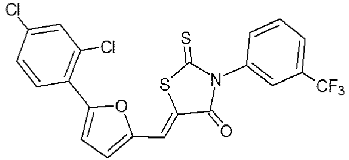 |
| 36 | 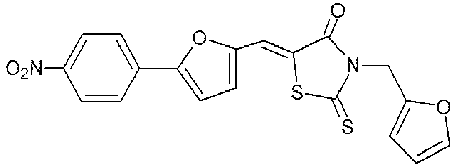 |
| 37 | 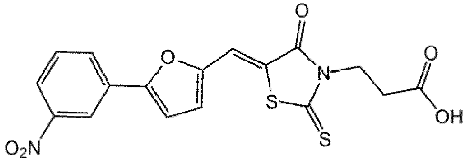 |
| 38 | 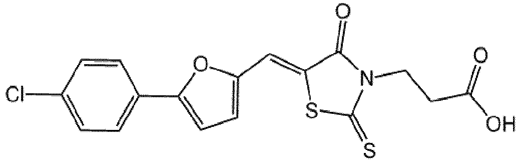 |
| 39 | 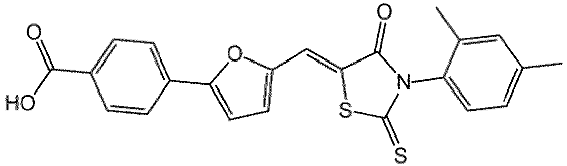 |
| 40 | 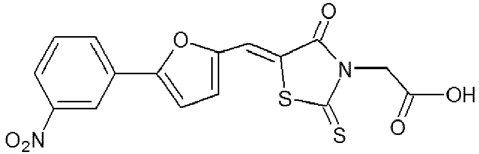 |
| 41 | 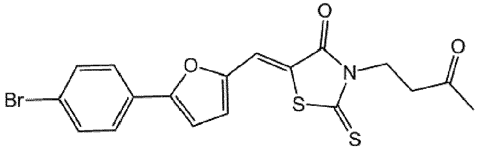 |

According to one embodiment, the compound may rescue the loss of cartilage matrix.

According to one embodiment, the compound may alleviate synovitis.

The activity of the compound of the present disclosure for the amelioration or treatment of osteoarthritis was confirmed through a novel animal model of osteoarthritis induced by overexpressing ZIP8 in mouse cartilage and joint tissues. ZIP8 is a transport protein (carrier) that is located in the membrane of chondrocytes and promotes the import of zinc ions. When the expression of ZIP8 increases, the import of zinc ions into chondrocytes may increase and the expression of cartilage matrix degrading enzymes may increase, resulting in osteoarthritis. The novel animal model shows loss of cartilage matrix and synovitis due to overexpression of ZIP8, and thus may be effectively used to confirm the effect of candidate drug on osteoarthritis treatment or the effect of ameliorating osteoarthritis caused by increased expression of ZIP8 (Regulation of the catabolic cascade in osteoarthritis by the zinc-ZIP8-MTF1 axis. Cell 156, 730-743 (2014)).

According to one example of the present disclosure, as a result of administering each of compounds 1 to 3 (AVIX-7, MA3001, and MA6004) to a ZIP8-overexpressing animal model, it was confirmed that each of the compounds exhibited the activity of rescuing the loss of cartilage matrix and alleviating synovitis. Accordingly, in the present disclosure, compounds 1 to 3 and the compound represented by Formula 1 that share structural characteristics therewith may be effectively used for the prevention or treatment of osteoarthritis.

According to one embodiment, the osteoarthritis may be arthritis caused by increased expression of ZIP8. Since the compound may inhibit cartilage destruction caused by zinc imported by ZIP8 and inflammation in joints, it may exhibit a therapeutic effect against osteoarthritis caused by increased expression of ZIP8.

For the treatment of osteoarthritis, the pharmaceutical composition according to one embodiment of the present disclosure may be used alone or in combination with surgery, hormonal therapy, drug therapy, radiotherapy, and/or methods of using biological response modifiers.

The compound represented by Formula 1 may be produced by a method known to those skilled in the art. For example, it may be produced according to the method described in Korean Patent No. 10-1159000 or US Patent No. 8,759,536.

The term "rhodanine derivative" refers to a compound having a rhodanine structure. Since the compound represented by Formula 1 has a rhodanine structure, it may be referred to as a rhodanine derivative in the present specification.

The compound represented by Formula 1 may be produced as a pharmaceutically acceptable salt or solvate according to a conventional method known in the art.

The pharmaceutically acceptable salt is advantageously an acid addition salt formed with a free acid. The acid addition salt is prepared in a conventional manner, for example, by dissolving the compound in excess aqueous acid solution and precipitating the salt with a water-miscible organic solvent such as methanol, ethanol, acetone or acetonitrile. Equimolar amounts of the compound and acid may also be heated in water or an alcohol (e.g., glycol monomethyl ether) and then the mixture may be evaporated to dryness, or the precipitated salt may be filtered off with suction.

As the free acids, organic acids and inorganic acids may be used. Examples of the inorganic acid include hydrochloric acid, phosphoric acid, sulfuric acid, nitric acid, tartaric acid, and the like, and examples of the organic acids include methanesulfonic acid, p-toluenesulfonic acid, acetic acid, trifluoroacetic acid, citric acid, maleic acid, succinic acid, oxalic acid, benzoic acid, tartaric acid, fumaric acid, mandelic acid, propionic acid, lactic acid, glycolic acid, gluconic acid, galacturonic acid, glutamic acid, glutaric acid, glucuronic acid, aspartic acid, ascorbic acid, carbonic acid, vanillic acid, hydroiodic acid, and the like.

In addition, a pharmaceutically acceptable metal salt may be prepared using bases. An alkali metal or alkaline earth metal salt is obtained, for example, by dissolving the compound in an excessive amount of alkali metal or alkaline earth metal hydroxide solution, filtering off an undissolved compound salt and evaporating the filtrate to dryness. At this time, as the metal salt, a sodium, potassium or calcium salt is a pharmaceutically suitable salt. In addition, the corresponding silver salt is obtained by reaction of an alkali metal salt or alkaline earth metal salt with a suitable silver salt (e.g., silver nitrate).

Pharmaceutically acceptable salts of the compound represented by Formula 1 include salts of acidic or basic groups that may exist in the rhodanine derivative, unless otherwise specified. Examples of the pharmaceutically acceptable salts include sodium, calcium and potassium salts of a hydroxyl group, and examples of other pharmaceutically acceptable salts of an amino group include hydrobromide, sulfate, hydrogen sulfate, phosphate, hydrogen phosphate, dihydrogen phosphate, acetate, succinate, citrate, tartrate, lactate, mandelate, methanesulfonate (mesylate) and para-toluenesulfonate (tosylate) salts. The salts may be prepared using a salt preparation method or process known in the art.

The pharmaceutical composition of the present disclosure may contain a pharmaceutically acceptable carrier. Examples of the pharmaceutically acceptable carrier that is contained in the pharmaceutical composition of the present disclosure include, but are not limited to, lactose, dextrose, sucrose, sorbitol, mannitol, starch, gum acacia, calcium phosphate, alginate, gelatin, calcium silicate, microcrystalline cellulose, polyvinyl pyrrolidone, cellulose, water, syrup, methyl cellulose, methyl hydroxybenzoate, propyl hydroxylbenzoate, talc, magnesium stearate, and mineral oil, which are commonly used in the manufacture of medicaments. The pharmaceutical composition of the present disclosure may further contain, in addition to the above components, a lubricant, a wetting agent, a sweetening agent, a flavoring agent, an emulsifier, a suspending agent, a preservative, etc. Suitable pharmaceutically acceptable carriers and formulations are described in detail in Remington's Pharmaceutical Sciences (22nd ed., 2013) .

The pharmaceutical composition of the present disclosure may contain various bases and/or additives necessary and suitable for formulation thereof, and may be prepared to further contain known components such as a nonionic surfactant, a silicone polymer, an extender pigment, a fragrance, an antiseptic agent, a disinfectant, an oxidation stabilizer, an organic solvent, an ionic or nonionic thickener, a softener, an antioxidant, a free radical destruction agent, an opacifier, a stabilizer, an emollient, silicone, α-hydroxy acid, an antifoaming agent, a moisturizer, a vitamin, an insect repellent, a fragrance, a preservative, a surfactant, an anti-inflammatory agent, a substance P antagonist, a filler, a polymer, a propellant, a basifying or acidifying agent, or a coloring agent, within a range that does not impair the effect thereof.

A suitable dose of the pharmaceutical composition of the present disclosure may vary depending on factors such as the method of formulation, the mode of administration, the patient's age, body weight, sex, disease condition and diet, the duration of administration, the route of administration, excretion rate, and response sensitivity. The dose of the pharmaceutical composition of the present disclosure may be 0.001 to 1,000 mg/kg for adults.

The pharmaceutical composition of the present disclosure may be administered parenterally. For example, the pharmaceutical composition may be administered through a method such as intra-articular injection, subcutaneous injection, intravenous injection, intramuscular injection or intrathoracic injection, but the administration method is not limited thereto.

For formulation into a parenteral dosage form, for example, the pharmaceutical composition of the present disclosure may be mixed with a stabilizer or a buffer in water to prepare a solution or a suspension, which is then prepared into an ampoule or vial unit dosage form. In addition, the composition may be sterilized, or may further contain an adjuvant, such as a preservative, a stabilizer, a hydrating agent or an emulsification accelerator, a salt for controlling osmotic pressure, or a buffer, and other therapeutically beneficial substances, and may be formulated according to a conventional method.

Another aspect of the present disclosure provides a method for treating osteoarthritis, the method including a step of administering to an osteoarthritis patient a composition containing, as an active ingredient, a compound selected from the group consisting of the compounds represented by Formula 1.

Description of the compound represented by Formula 1 is as described above.

According to one embodiment, the compound represented by Formula 1 may be a compound selected from the group consisting of: 1) 3-{5-[5-(4-chloro-phenyl)-furan-2-ylmethylene]-4-oxo-2-thioxo-thiazolidin-3-yl}-propionic acid; 2) 4-(5-((5-(4-(isopropoxycarbonyl)phenyl)furan-2-yl)methylene)-4-oxo-2-thioxothiazolidin-3-yl)butanoic acid; 3) 3-(5-((5-(4-chlorophenyl)furan-2-yl)methyl)-2,4-dioxothiazolidin-3-yl)propionic acid; 4) 3-(2,4-dimethylphenyl)-5-[5-(3-nitrophenyl)-furan-2-ylmethylene]-2-thioxothiazolidine-4-one; 5) 5-[5-(4-morpholine-4-yl-3-nitrophenyl)-furan-2-ylmethylene]-2-thioxothiazolidine-4-one; 6) 5-[5-(4-oxo-2-thioxothiazolidine-5-ylidenemethyl)-furan-2-yl]-isoindole-1,3-dione; 7) 3-(2-methanesulfonylethyl)-5-[5-(3-nitrophenyl)-furan-2-ylmethylene]-2-thioxothiazolidine-4-one; 8) 5-[5-(4-nitrophenyl)-furan-2-ylmethylene]-3-(tetrahydrofuran-2-ylmethyl)-2-thioxothiazolidine-4-one; 9) 3-(4-diethylaminophenyl)-5-[5-(4-nitrophenyl)-furan-2-ylmethylene]-2-thioxothiazolidine-4-one; 10) 5-[5-(2,4-dichlorophenyl)-furan-2-ylmethylene]-3-(3-fluorophenyl)-2-thioxothiazolidine-4-one; 11) 3-phenethyl-5-(5-phenyl-furan-2-ylmethylene)-2-thioxothiazolidine-4-one; 12) 5-[5-(3-chloro-4-methylphenyl)-furan-2-ylmethylene]-3-ethyl-2-thioxothiazolidine-4-one; 13) 5-(naphthalen-1-yl)furan-2-ylmethylene-3-(2-oxopropyl)-2-thioxothiazolidine-4-one; 14) 3-cyclohexyl-5-[5-(2-nitrophenyl)-furan-2-ylmethylene]-2-thioxothiazolidine-4-one; 15) 3-(3-fluorophenyl)-5-[5-(4-nitrophenyl)-furan-2-ylmethylene]-2-thioxothiazolidine-4-one; 16) 3-(1,1-dioxotetrahydrothiophen-3-yl)-5-[5-(2-nitrophenyl)-furan-2-ylmethylene]-2-thioxothiazolidine-4-one; 17) 5-[5-(2-nitrophenyl)-furan-2-ylmethylene]-3-(4-oxopentyl)-2-thioxothiazolidine-4-one; 18) 5-[5-(2-bromo-5-nitrophenyl)furan-2-ylmethylene]-3-(4-iodophenyl)-2-thioxothiazolidine-4-one; 19) 5-[5-(2-chloro-4-nitrophenyl)furan-2-ylmethylene]-2-thioxo-3-(3-trifluoromethylphenyl)-thiazolidine-4-one; 20) 3-benzyl-5-[5-(4-ethoxy-2-nitrophenyl)-furan-2-ylmethylene]-2-thioxothiazolidine-4-one; 21) {5-[5-(2-methyl-5-nitrophenyl)furan-2-ylmethylene]-4-oxo-2-thioxothiazolidine-3-yl}acetic acid; 22) {5-[5-(4-ethoxy-2-nitrophenyl)-furan-2-ylmethylene]-4-oxo-2-thioxothiazolidine-3-yl}-acetic acid; 23) {5-[5-(4-methoxy-2-nitrophenyl)-furan-2-ylmethylene]-4-oxo-2-thioxothiazolidine-3-yl}acetic acid; 24) 3-(4-nitrophenyl)-5-[5-(4-nitrophenyl)-furan-2-ylmethylene]-2-thioxothiazolidine-4-one; 25) 4-[5-(4-oxo-2-thioxothiazolidine-5-ylidenemethyl)-furan-2-yl]-benzoicisopropylester; 26) 5-[5-(2,5-dichlorophenyl)furan-2-ylmethylene]-3-(1,5-dimethyl-3-oxo-2-phenyl-2,3-dihydro-1H-pyrazole-4-yl)-2-thioxothiazolidine-4-one; 27) 5-[5-(1-oxo-1,3-dihydroisobenzofuran-5-yl)-furan-2-ylmethylene]-2-thioxothiazolidine-4-one; 28) 3-(furan-2-ylmethyl)-5-[5-(2-nitrophenyl)furan-2-ylmethylene]-2-thioxothiazolidine-4-one; 29) 5-[5-(2-nitrophenyl)-furan-2-ylmethylene]-3-phenethyl-2-thioxothiazolidine-4-one; 30) 3-(3-chlorophenyl)-5-[5-(2,3-dichlorophenyl)-furan-2-ylmethylene]-2-thioxothiazolidine-4-one; 31) 2-chloro-5-[5-(4-oxo-2-thioxothiazolidine-5-ylidenemethyl)-furan-2-yl]-benzoicpropylester; 32) 4-{5-[5-(3,4-dichlorophenyl)-furan-2-ylmethylene]-4-oxo-2-thioxothiazolidine-3-yl}-buyric acid; 33) 2-{5-[5-(4-nitrophenyl)-furan-2-ylmethylene]-4-oxo-2-thioxothiazolidine-3-yl}-succinic acid; 34) 5-[5-(4-chlorophenyl)furan-2-ylmethylene]-3-(furan-2-ylmethyl)-2-thioxothiazolidine-4-one; 35) 5-[5-(2,4-dichlorophenyl)furan-2-ylmethylene]-2-thioxo-3-(3-trifluoromethylphenyl)-thiazolidine-4-one; 36) 3-(furan-2-ylmethyl)-5-[5-(4-nitrophenyl)furan-2-ylmethylene]-2-thioxothiazolidine-4-one; 37) 3-{5-[5-(3-nitrophenyl)furan-2-ylmethylene]-4-oxo-2-thioxothiazolidine-3-yl}-propionic acid; 38) 3-{5-[5-(4-chlorophenyl)furan-2-ylmethylene]-4-oxo-2-thioxothiazolidine-3-yl}-propionic acid; 39) 4-{5-[3-(2,4-dimethylphenyl)-4-oxo-2-thioxothiazolidin-5-ylidenemethyl]-furan-2-yl}benzoic acid; 40) {5-[5-(3-nitrophenyl)furan-2-ylmethylene]-4-oxo-2-thioxothiazolidin-3-yl}acetic acid; and 41) 5-[5-(4-bromophenyl)-furan-2-ylmethylene]-3-(3-oxobutyl)-2-thioxothiazolidine-4-one.

The compound represented by Formula 1 according to the present disclosure has an excellent effect of ameliorating symptoms of osteoarthritis, and thus may be effectively used to treat osteoarthritis patients.

Still another aspect of the present disclosure provides a food composition for preventing or ameliorating osteoarthritis, the food composition containing the compound represented by Formula 1.

Description of the compound represented by Formula 1 is as described above.

According to one embodiment, the compound represented by Formula 1 may be a compound selected from the group consisting of: 1) 3-{5-[5-(4-chloro-phenyl)-furan-2-ylmethylene]-4-oxo-2-thioxo-thiazolidin-3-yl}-propionic acid; 2) 4-(5-((5-(4-(isopropoxycarbonyl)phenyl)furan-2-yl)methylene)-4-oxo-2-thioxothiazolidin-3-yl)butanoic acid; 3) 3-(5-((5-(4-chlorophenyl)furan-2-yl)methyl)-2,4-dioxothiazolidin-3-yl)propionic acid; 4) 3-(2,4-dimethylphenyl)-5-[5-(3-nitrophenyl)-furan-2-ylmethylene]-2-thioxothiazolidine-4-one; 5) 5-[5-(4-morpholine-4-yl-3-nitrophenyl)-furan-2-ylmethylene]-2-thioxothiazolidine-4-one; 6) 5-[5-(4-oxo-2-thioxothiazolidine-5-ylidenemethyl)-furan-2-yl]-isoindole-1,3-dione; 7) 3-(2-methanesulfonylethyl)-5-[5-(3-nitrophenyl)-furan-2-ylmethylene]-2-thioxothiazolidine-4-one; 8) 5-[5-(4-nitrophenyl)-furan-2-ylmethylene]-3-(tetrahydrofuran-2-ylmethyl)-2-thioxothiazolidine-4-one; 9) 3-(4-diethylaminophenyl)-5-[5-(4-nitrophenyl)-furan-2-ylmethylene]-2-thioxothiazolidine-4-one; 10) 5-[5-(2,4-dichlorophenyl)-furan-2-ylmethylene]-3-(3-fluorophenyl)-2-thioxothiazolidine-4-one; 11) 3-phenethyl-5-(5-phenyl-furan-2-ylmethylene)-2-thioxothiazolidine-4-one; 12) 5-[5-(3-chloro-4-methylphenyl)-furan-2-ylmethylene]-3-ethyl-2-thioxothiazolidine-4-one; 13) 5-(naphthalen-1-yl)furan-2-ylmethylene-3-(2-oxopropyl)-2-thioxothiazolidine-4-one; 14) 3-cyclohexyl-5-[5-(2-nitrophenyl)-furan-2-ylmethylene]-2-thioxothiazolidine-4-one; 15) 3-(3-fluorophenyl)-5-[5-(4-nitrophenyl)-furan-2-ylmethylene]-2-thioxothiazolidine-4-one; 16) 3-(1,1-dioxotetrahydrothiophen-3-yl)-5-[5-(2-nitrophenyl)-furan-2-ylmethylene]-2-thioxothiazolidine-4-one; 17) 5-[5-(2-nitrophenyl)-furan-2-ylmethylene]-3-(4-oxopentyl)-2-thioxothiazolidine-4-one; 18) 5-[5-(2-bromo-5-nitrophenyl)furan-2-ylmethylene]-3-(4-iodophenyl)-2-thioxothiazolidine-4-one; 19) 5-[5-(2-chloro-4-nitrophenyl)furan-2-ylmethylene]-2-thioxo-3-(3-trifluoromethylphenyl)-thiazolidine-4-one; 20) 3-benzyl-5-[5-(4-ethoxy-2-nitrophenyl)-furan-2-ylmethylene]-2-thioxothiazolidine-4-one; 21) {5-[5-(2-methyl-5-nitrophenyl)furan-2-ylmethylene]-4-oxo-2-thioxothiazolidine-3-yl}acetic acid; 22) {5-[5-(4-ethoxy-2-nitrophenyl)-furan-2-ylmethylene]-4-oxo-2-thioxothiazolidine-3-yl}-acetic acid; 23) {5-[5-(4-methoxy-2-nitrophenyl)-furan-2-ylmethylene]-4-oxo-2-thioxothiazolidine-3-yl}acetic acid; 24) 3-(4-nitrophenyl)-5-[5-(4-nitrophenyl)-furan-2-ylmethylene]-2-thioxothiazolidine-4-one; 25) 4-[5-(4-oxo-2-thioxothiazolidine-5-ylidenemethyl)-furan-2-yl]-benzoicisopropylester; 26) 5-[5-(2,5-dichlorophenyl)furan-2-ylmethylene]-3-(1,5-dimethyl-3-oxo-2-phenyl-2,3-dihydro-1H-pyrazole-4-yl)-2-thioxothiazolidine-4-one; 27) 5-[5-(1-oxo-1,3-dihydroisobenzofuran-5-yl)-furan-2-ylmethylene]-2-thioxothiazolidine-4-one; 28) 3-(furan-2-ylmethyl)-5-[5-(2-nitrophenyl)furan-2-ylmethylene]-2-thioxothiazolidine-4-one; 29) 5-[5-(2-nitrophenyl)-furan-2-ylmethylene]-3-phenethyl-2-thioxothiazolidine-4-one; 30) 3-(3-chlorophenyl)-5-[5-(2,3-dichlorophenyl)-furan-2-ylmethylene]-2-thioxothiazolidine-4-one; 31) 2-chloro-5-[5-(4-oxo-2-thioxothiazolidine-5-ylidenemethyl)-furan-2-yl]-benzoicpropylester; 32) 4-{5-[5-(3,4-dichlorophenyl)-furan-2-ylmethylene]-4-oxo-2-thioxothiazolidine-3-yl}-butanoic acid; 33) 2-{5-[5-(4-nitrophenyl)-furan-2-ylmethylene]-4-oxo-2-thioxothiazolidine-3-yl}-succinic acid; 34) 5-[5-(4-chlorophenyl)furan-2-ylmethylene]-3-(furan-2-ylmethyl)-2-thioxothiazolidine-4-one; 35) 5-[5-(2,4-dichlorophenyl)furan-2-ylmethylene]-2-thioxo-3-(3-trifluoromethylphenyl)-thiazolidine-4-one; 36) 3-(furan-2-ylmethyl)-5-[5-(4-nitrophenyl)furan-2-ylmethylene]-2-thioxothiazolidine-4-one; 37) 3-{5-[5-(3-nitrophenyl)furan-2-ylmethylene]-4-oxo-2-thioxothiazolidine-3-yl}-propionic acid; 38) 3-{5-[5-(4-chlorophenyl)furan-2-ylmethylene]-4-oxo-2-thioxothiazolidine-3-yl}-propionic acid; 39) 4-{5-[3-(2,4-dimethylphenyl)-4-oxo-2-thioxothiazolidin-5-ylidenemethyl]-furan-2-yl}benzoic acid; 40) {5-[5-(3-nitrophenyl)furan-2-ylmethylene]-4-oxo-2-thioxothiazolidin-3-yl}acetic acid; and 41) 5-[5-(4-bromophenyl)-furan-2-ylmethylene]-3-(3-oxobutyl)-2-thioxothiazolidine-4-one.

The compound represented by Formula 1 according to the present disclosure may rescue the loss of cartilage matrix and alleviate symptoms of synovitis, and thus the food composition containing the same may prevent the occurrence of symptoms of osteoarthritis and ameliorate symptoms of osteoarthritis that have already occurred.

The food composition of the present disclosure may be prepared by adding raw materials and components, which are commonly added in the art, in addition to containing, as an active ingredient, the compound represented by Formula 1. In addition, the food composition of the present disclosure may contain, as additional ingredients, various flavorings or natural carbohydrates, like conventional food composition.

According to one embodiment of the present disclosure, the natural carbohydrates may be conventional sugars, such as monosaccharides (e.g., glucose, fructose, etc.), disaccharides (e.g., maltose, sucrose, etc.), and polysaccharides (e.g., dextrin, cyclodextrin, etc.), and sugar alcohols such as xylitol, sorbitol, or erythritol. Examples of the flavorings may include natural flavorings (thaumatin), stevia extracts (e.g., rebaudioside A, glycyrrhizin, etc.) and/or synthetic flavorings (saccharin, aspartame, etc.).

The food composition of the present disclosure may be formulated as a food composition further containing one or more food-acceptable or pharmaceutically acceptable carriers in addition to the above-described active ingredient. The food composition may be formulated in the form of tablet, capsule, powder, granule, liquid, pill, solution, syrup, juice, suspension, emulsion, or drop. For example, for formulation in the form of tablet or capsule, the active ingredient may be combined with an oral, non-toxic pharmaceutically acceptable inert carrier such as ethanol, glycerol, or water.

The food composition of the present disclosure may contain a vitamin mixture consisting of vitamin A acetate, vitamin E, vitamin B1, vitamin B2, vitamin B6, vitamin B12, vitamin C, biotin, nicotinic acid amide, folic acid, and calcium pantothenate, and one or more minerals such as ferrous sulfate, zinc oxide, magnesium carbonate, potassium phosphate monobasic, potassium phosphate dibasic, potassium citrate, calcium carbonate, and magnesium chloride, which may be commonly added in the art.

If necessary, suitable binders, lubricants, disintegrants and coloring agents may also be included as a mixture. Examples of suitable binders may include natural sugars such as starch, gelatin, glucose or beta-lactose, natural and synthetic gums such as corn sweeteners, acacia, tragacanth or sodium oleate, sodium stearate, magnesium stearate, sodium benzoate, sodium acetate, sodium chloride, and the like. Examples of the disintegrants may include starch, methyl cellulose, agar, bentonite, xanthan gum, and the like.

These components may be used independently or in combination, and the proportion of these additives may be selected within a range of from 0 to about 20 parts by weight based on 100 parts by weight of the food composition of the present disclosure, but is not limited thereto.

Meanwhile, it is possible to prepare a variety of foods by applying methods for preparing various formulations, known to those skilled in the art, to the food composition of the present disclosure. For example, the food composition of the present disclosure may be prepared as a common health functional food formulation such as a beverage, a pill, or a powder, but is not limited thereto.

The food composition of the present disclosure has an excellent effect of ameliorating symptoms caused by osteoarthritis, and thus may be effectively used for the prevention or amelioration of osteoarthritis.

### Advantageous Effects

The compound of the present disclosure has an excellent effect of rescuing the loss of cartilage matrix and alleviating synovitis, and thus may be effectively used for the prevention, amelioration or treatment of osteoarthritis.

### Brief Description of Drawings

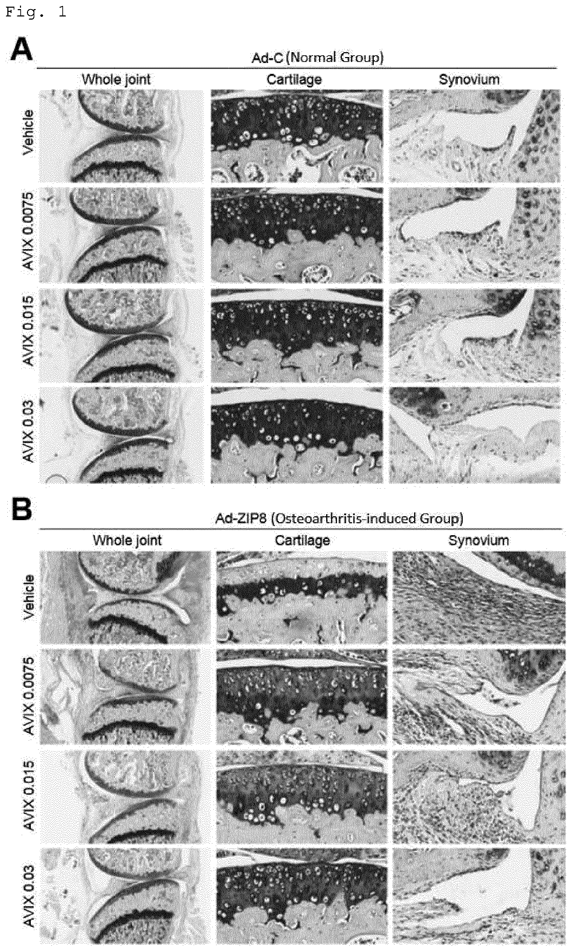

FIG. 1 depicts tissue images showing the results of observing the therapeutic effect of AVIX-7 on osteoarthritis induced by overexpressing the ZIP8 gene. Each of a vehicle and different concentrations of AVIX-7 was administered to mice, and whole joint, cartilage and synovium tissues in the mice were observed by staining with safranin-O. Ad-C in FIG. 1A is a normal group, and Ad-ZIP8 in FIG. 1B is a mouse group with osteoarthritis induced by ZIP8 overexpression. The vehicle is a control containing no drug.

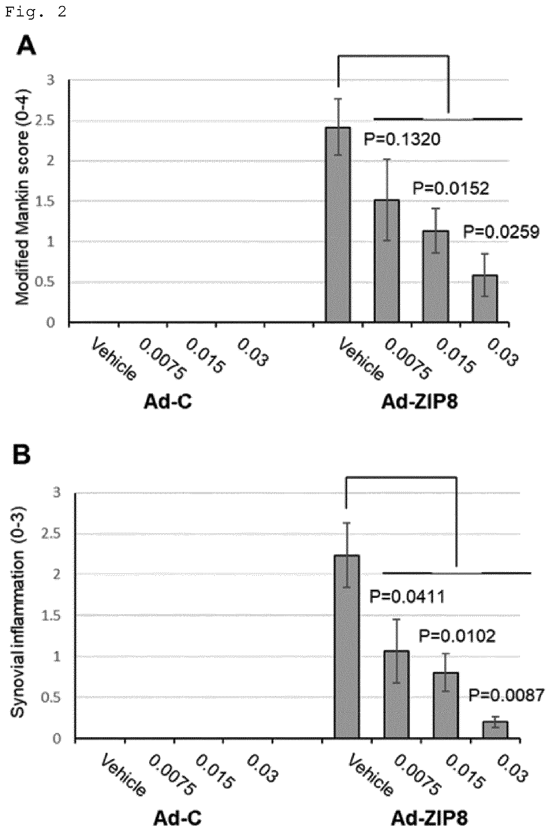

FIG. 2A is a graph showing the degree of cartilage destruction graded according to the modified Mankin score in the osteoarthritis-induced mice to which AVIX-7 was administered. FIG. 2B shows the results of evaluating the degree of synovitis after the administration of different concentrations of AVIX-7 to mice with osteoarthritis induced by ZIP8 overexpression.

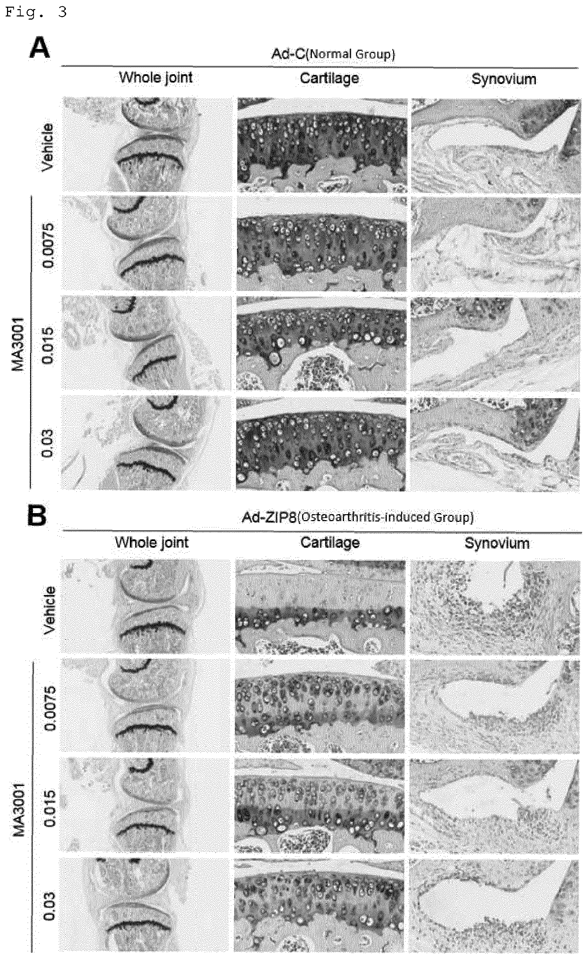

FIG. 3 depicts tissue images showing the results of observing the therapeutic effect of MA3001 on osteoarthritis induced by expressing the ZIP8 gene. Specifically, FIG. 3 shows whole joint, cartilage and synovium tissues observed by staining with safranin-O after administration of each of a vehicle and different concentrations of a compound (MA3001) according to one embodiment of the present disclosure to osteoarthritis-induced mice.

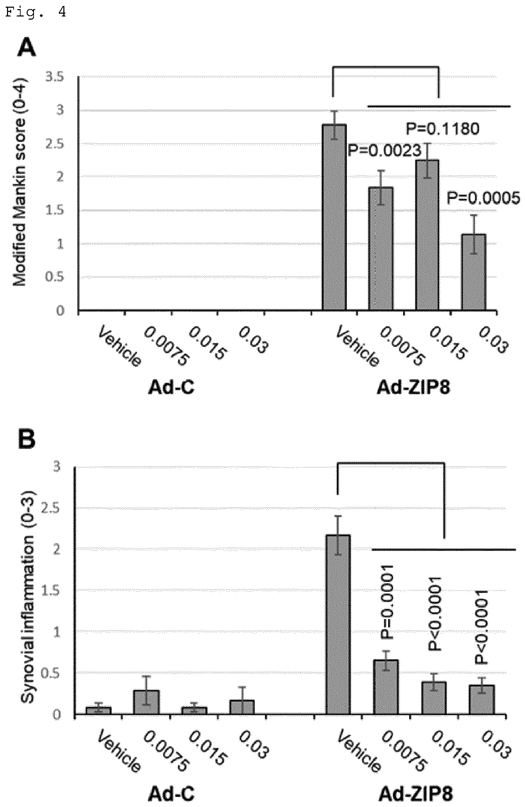

FIG. 4A is a graph showing the degree of cartilage destruction graded according to the modified Mankin score in the osteoarthritis-induced mice to which MA3001 was administered. FIG. 4B shows the results of evaluating the degree of synovitis after by the administration of different concentrations of MA3001 to mice with osteoarthritis induced by ZIP8 overexpression.

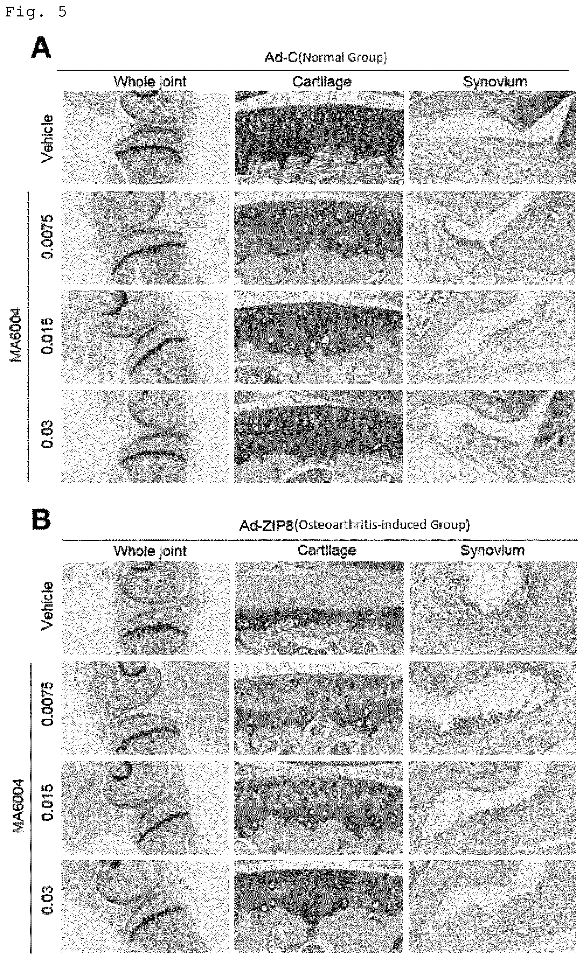

FIG. 5 depicts tissue images showing the results of observing the therapeutic effect of MA6004 on osteoarthritis induced by expressing the ZIP8 gene. Specifically, FIG. 5 shows whole joint, cartilage and synovium tissues observed by staining with safranin-O after administration of each of a vehicle (A) and different concentrations of a compound (AVIX-7) according to one embodiment of the present disclosure to osteoarthritis-induced mice.

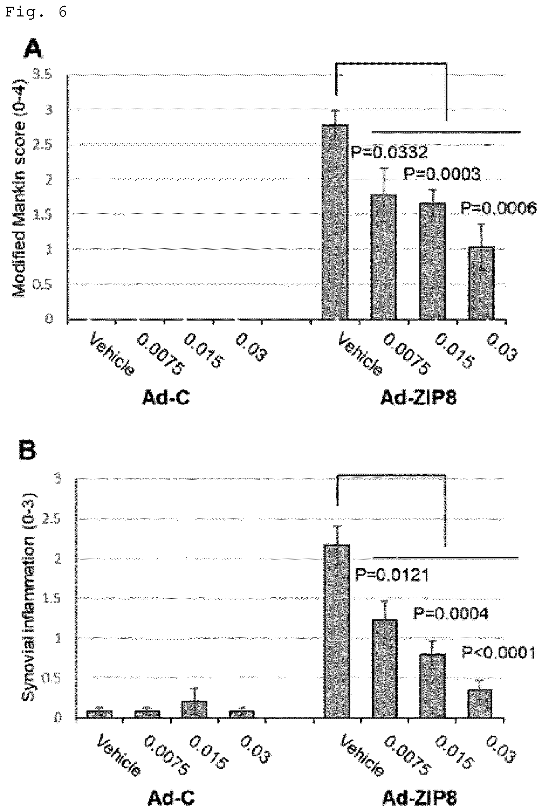

FIG. 6A is a graph showing the degree of cartilage destruction graded according to the modified Mankin score in the osteoarthritis-induced mice to which MA6004 was administered. FIG. 6B shows the results of evaluating the degree of synovitis after the administration of different concentrations of MA6004 to mice with osteoarthritis induced by ZIP8 overexpression.

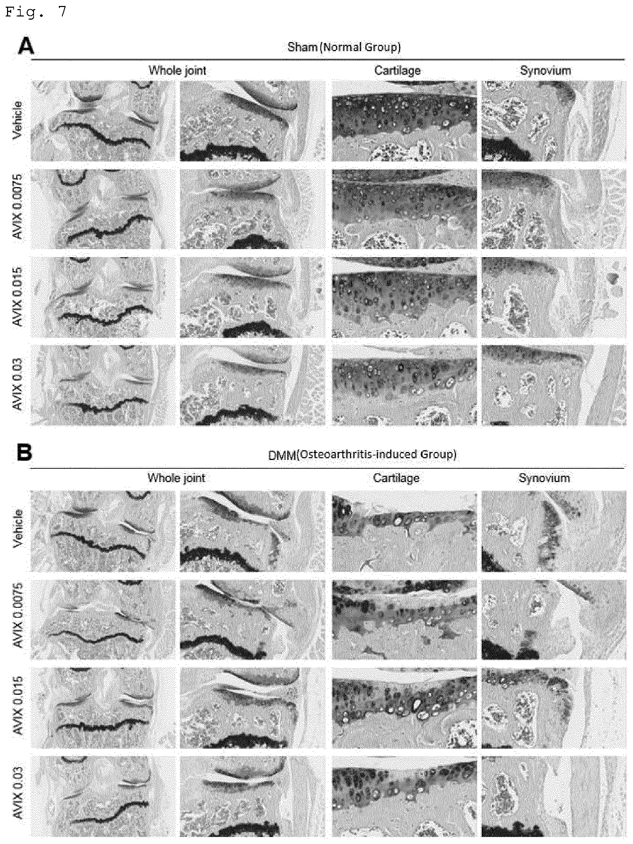

FIG. 7 shows the results of observing the therapeutic effect of AVIX-7 after administration of AVIX-7 to mice with osteoarthritis induced by destabilization of the medial meniscus (DMM). Specifically, FIGS. 7A and 7B are images showing the results of observing whole joint, cartilage and synovium tissues by staining with safranin-O after administration of different concentrations of AVIX-7 to normal mice (Sham) and mice with osteoarthritis induced by DMM, respectively.

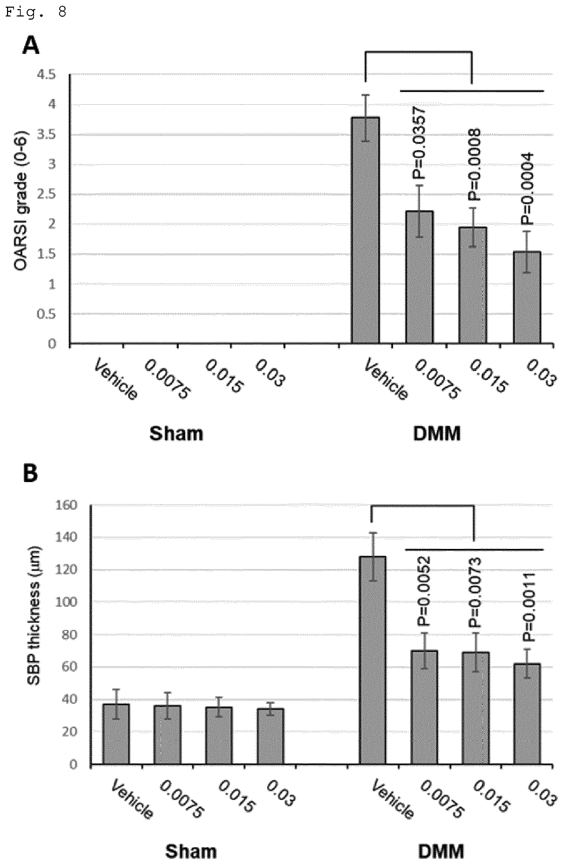

FIG. 8A is a graph showing osteoarthritis graded according to the OARSI grading method after administration of different concentrations of AVIX-7 to mice with osteoarthritis induced by DMM, and FIG. 8B is a graph showing the results of measuring the subchondral bone plate thickness after administration of different concentrations of AVIX-7 to mice with osteoarthritis induced by DMM.

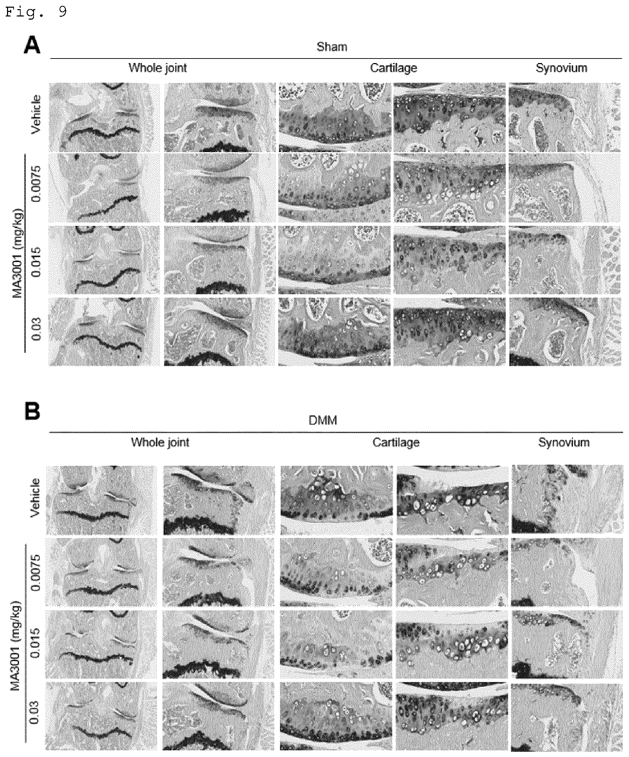

FIG. 9 shows the results of observing the therapeutic effect of MA3001 on osteoarthritis induced by destabilization of the medial meniscus (DMM). Specifically, FIGS. 9A and 9B are images showing the results of observing whole joint, cartilage and synovium tissues by staining with safranin-O after administration of different concentrations of MA3001 to normal mice (Sham) and mice with osteoarthritis induced by DMM, respectively.

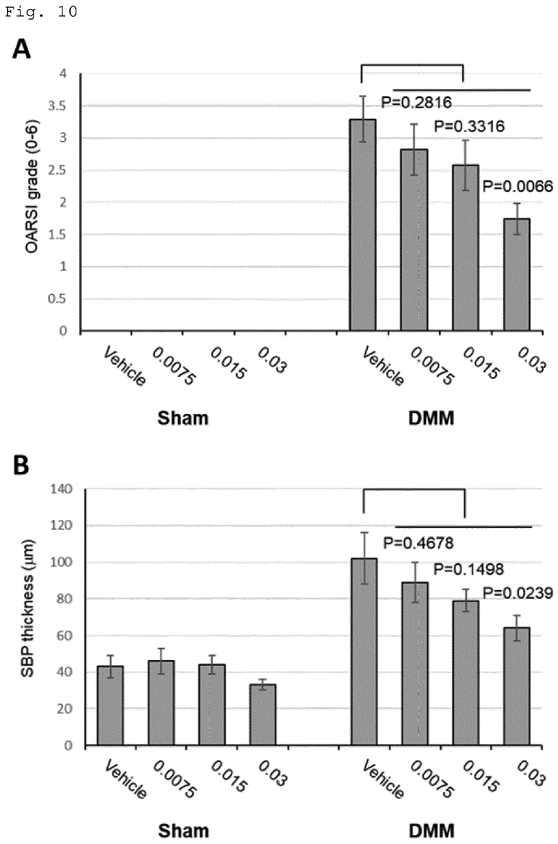

FIG. 10A is a graph showing osteoarthritis graded according to the OARSI grading method after administration of different concentrations of MA3001 to mice with osteoarthritis induced by DMM, and FIG. 10B is a graph showing the results of measuring the subchondral bone plate thickness after administration of different concentrations of MA3001 to mice with osteoarthritis induced by DMM.

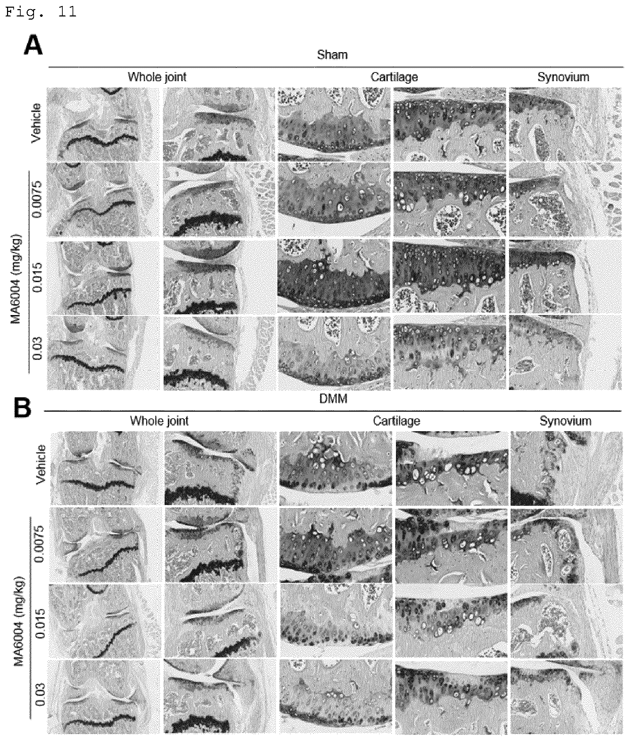

FIG. 11 shows the results of observing the therapeutic effect of MA6004 on osteoarthritis induced by destabilization of the medial meniscus (DMM). Specifically, FIGS. 11A and 11B are images showing the results of observing whole joint, cartilage and synovium tissues by staining with safranin-O after administration of different concentrations of MA6004 to normal mice (Sham) and mice with osteoarthritis induced by DMM, respectively.

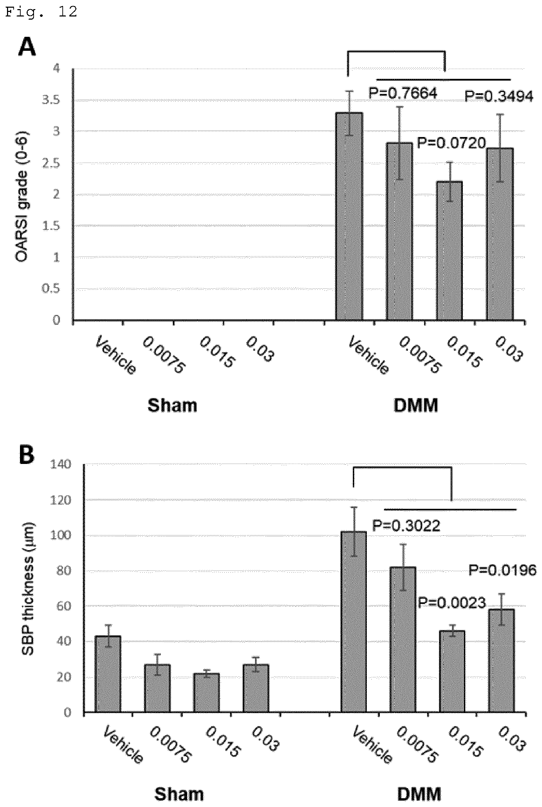

FIG. 12A is a graph showing osteoarthritis graded according to the OARSI grading method after administration of different concentrations of MA6004 to mice with osteoarthritis induced by DMM, and FIG. 12B is a graph showing the results of measuring the subchondral bone plate thickness after administration of different concentrations of MA6004 to mice with osteoarthritis induced by DMM.

### Mode for Invention

Hereinafter, the present disclosure will be described in detail with reference to one or more examples. However, these examples serve merely to illustrate the present disclosure, and the scope of the present disclosure is not limited by these examples.

### Example 1. Induction of Osteoarthritis by ZIP8 Overexpression in Mice and Treatment with Therapeutic Agent

Osteoarthritis-induced mice were prepared by overexpressing the zinc importer ZIP8 in the cartilage and joint tissues of mice.

Specifically, the cartilage and joint tissues of about 12-week-old male C57/BL6 mice were injected intra-articularly with a normal control adenovirus (Ad-C) or a ZIP8-expressing adenovirus (Ad-Slc39a8). A vehicle group was treated only with a vehicle without a vector. Each of the adenoviruses was injected at a concentration of 1X10⁹ PFU a total of three times (once a week).

Meanwhile, in order to evaluate the effect of each of the therapeutic agents AVIX-7, MA3001 and MA6004 on the amelioration of osteoarthritis induced by ZIP8 overexpression, AVIX-7, MA3001 or MA6004 was co-injected into the joint when each adenovirus was injected.

The structure of AVIX-7 corresponds to compound 1 in Table 1 above, the structure of MA3001 corresponds to compound 2 in Table 1 above, and the structure of MA6004 corresponds to compound 3 in Table 1 above.

Each of the therapeutic agents was used at doses of 0.0075 mg/kg, 0.015 mg/kg and 0.03 mg/kg mouse body weight. Each therapeutic agent was administered by injection once a week, and the amount of each therapeutic agent administered at each time point was 10 µl. One week after the day of the last injection of a total of three injections, the mice were sacrificed and the degree of cartilage destruction and the degree of occurrence of synovitis were analyzed.

### Example 2. Induction of Osteoarthritis in Mice by DMM and Treatment with Therapeutic Agent

Mice with osteoarthritis induced by destabilization of the medial meniscus (DMM) were prepared. As a control group, a group (sham) whose meniscus was not damaged during surgery was used.

Specifically, osteoarthritis was induced by damaging the menisci of joints of about 12-week-old male C57/BL6 mice, and each therapeutic agent (AVIX-7, MA3001 or MA6004) was injected intra-articularly.

Each of the therapeutic agents was used at doses of 0.0075 mg/kg, 0.015 mg/kg and 0.03 mg/kg mouse body weight. Each therapeutic agent was administered by injection once a week, and the amount of each therapeutic agent administered at each time point was 10 µl. One week after the day of the last injection of a total of three injections, the mice were sacrificed and the effect of each therapeutic agent on the amelioration of osteoarthritis was evaluated.

### Example 3. Evaluation of Effect of Therapeutic Agent on Amelioration of Cartilage Destruction in Model of Osteoarthritis Induced by Gene Expression

**In** order to evaluate the therapeutic effect of AVIX-7, MA3001 or MA6004 on osteoarthritis in ZIP8-overexpressing mice, whole joint and cartilage tissues were stained with safranin-O and the degree of cartilage destruction was checked.

Referring to the vehicle group (administered with no drug) in FIGS. 1, 3 and 5, the safranin-O red stained area in the mice in which ZIP8 overexpression (Ad-Sic39a8) was induced decreased compared to that in the normal group (Ad-C) (The red color is indicated by a dark color in the black and white figures). This is because the whole joint was thinned and the cartilage matrix was lost, due to osteoarthritis. On the other hand, it was confirmed that in the group treated with AVIX-7, MA3001 or MA6004, the loss of the cartilage matrix was concentration-dependently rescued (see whole joint and cartilage in FIGS. 1, 3 and 5).

In addition, the degree of safranin-O staining (reflecting the degree of cartilage destruction) according to Table 2 below was observed and degraded according to the modified Mankin score (Mankin, Henry J., and Louis Lippiello. JBJS 52.3 (1970): 424-434.), and the grades were quantified graphically. The Mankin score is an index indicating the degree of arthritis and may be determined by the degree of safranin-O staining in mice, and the lower the Mankin score, the closer to normal. The modified Mankin score is shown in FIGS., 2A, 4A and 6A.

**[Table 2]**

| Degree of safranin-O staining | Modified Mankin score |
|---|---|
| Normal | 0 |
| Slightly decreased | 1 |
| Moderately decreased | 2 |
| Significantly decreased | 3 |
| Not stained | 4 |

As a result, it could be confirmed that, in the ZIP8-overexpressing mice, the modified Mankin score of the vehicle group was 2 or higher, but the Mankin score of the group treated with AVIX-7, MA3001 or MA6004 was lower than that of the vehicle group and also decreased in a concentration-dependent manner. Thus, it was confirmed that AVIX-7, MA3001 and MA6004 had the effect of ameliorating cartilage destruction caused by osteoarthritis (FIGS. 2A, 4A and 6A).

### Example 4. Evaluation of Effect of Ameliorating Synovitis

In order to evaluate the osteoarthritis amelioration effect of each of the therapeutic agents developed in the present disclosure, the ZIP8-overexpressing animal model was treated with AVIX-7, MA3001 or MA6004, and the cartilage and joint tissues of the animal model were stained with hematoxylin-eosin/safranin-O, and the presence or absence of synovitis was checked. The ZIP8-overexpressing animal model was prepared by the method described in Example 1.

Referring to the synovium and vehicle groups in FIGS. 1, 3 and 5, the penetration of inflammatory cells into the synovium in the ZIP8-overexpressing mice (Ad-Sic39a8) increased compared to that in the normal group (Ad-C). On the other hand, it was confirmed that the inflammatory response in the group treated with AVIX-7, MA3001 or MA6004 was alleviated (see FIGS. 1, 3 and 5).

In addition, the degree of synovitis was scored according to "Synovitis score: discrimination between chronic low-grade and high-grade synovitis. Histopathology 49, 358-364" described in Table 3 below, and was quantified graphically. The score of synovitis is shown in FIGS. 2B, 4B and 6B.

**[Table 3]**

| Degree of synovitis | Grade |
|---|---|
| Normal | 0 |
| Lymphocytes or plasma cells located around blood vessels | 1 |
| Significant amounts of lymphocytes or plasma cells, formation of some follicle-like aggregates | 2 |
| Formation of band of dense inflammatory infiltrates or significant amounts of large follicle-like aggregates | 3 |

Referring to FIGS. 2B, 4B and 6B, it was confirmed that, in the ZIP8-overexpressing mice, synovitis occurred in the cartilage and joint tissues, and the score of synovitis was high (2 or higher), but in the group treated with AVIX-7, MA3001 or MA6004, the score of synovitis significantly decreased, suggesting that AVIX-7, MA3001 or MA6004 had the effect of ameliorating synovitis symptoms.

### Example 5. Evaluation of Effect on Osteoarthritis Amelioration in Model of Osteoarthritis Induced by Destabilization of Medial Meniscus

In order to evaluate the effect of the compounds of the present disclosure on the amelioration of osteoarthritis, mice were subjected to destabilization of the medial meniscus (DMM) to induce osteoarthritis and were treated with AVIX-7, MA3001 or MA6004. In addition, the cartilage and joint tissues were stained with hematoxylin-eosin/safranin-O, and the effect of AVIX-7, MA3001 or MA6004 on the amelioration of osteoarthritis was evaluated pathologically. Following the staining, the state of wear of the joint surface and the presence of osteophytes were evaluated by the grading method suggested by OARSI (Osteoarthritis Research Society International). The OARSI grading method is described in Table 4 below.

**[Table 4]**

| Criteria | Subgrade | Grade |
|---|---|---|
| Surface intact and cartilage intact, no damage | No subgrade | 0 |
| Matrix: superficial zone intact, edema and/or fibrillation cells: proliferation (clusters), hypertrophy | Surface intact 1.0: Cells intact, 1.5: Cell death | 1 |
| Safranin 0 or toluidine blue depletion on upper one-third of cartilage (mid zone), chondron column disorientation | Surface discontinuity (rough) 2.0: Fibrillation through superficial zone, 2.5: Surface abrasion with matrix loss within superficial zone | 2 |
| safranin 0 or toluidine blue depletion on lower two-thirds of cartilage (deep zone) | Vertical fissures 3.0: Simple fissures. 3.5: Branched/complex fissures | 3 |
| | | |
| Cartilage matrix destruction, cyst formation within cartilage matrix | Erosion 4.0: Superficial zone delamination, 4.5: Midzone excavation | 4 |
| Articular surface is sclerotic bone or reparative tissue including fibrocartilage | Denudation 5.0: Bone surface intact, 5.5: Reparative tissue present | 5 |
| Bone remodeling; deformation of articular surface contour including microfracture and repair | Deformation 6.0: Joint margin osteophytes, 6.5: Joint margin and central osteophyte | 6 |

Referring to the vehicle group in FIGS. 7, 9 and 11, the penetration of inflammatory cells into the whole joint and cartilage tissues in the mice with osteoarthritis induced by DMM significantly increased compared to that in the normal group (Sham). However, it was confirmed that the inflammation response in the group treated with AVIX-7, MA3001 or MA6004 was alleviated (see the images in FIGS. 7B, 9B and 11B and the OARSI grade in FIGS. 8A, 10A and 12A).

In addition, as a result of measuring the thickness of the subchondral bone plate (SBP) in the mice with osteoarthritis induced by DMM, it was confirmed that the SBP thickness in the vehicle group to which no drug was administered significantly increased, but the SBP thickness in the group to which AVIX-7, MA3001 or MA6004 was administered decreased (see FIGS. 8B, 10B and 12B). From these results, it was confirmed that AVIX-7 MA3001 and MA6004 all have the effect of ameliorating osteoarthritis.

**So** far, the present disclosure has been described with reference to the embodiments. Those of ordinary skill in the art to which the present disclosure pertains will appreciate that the present disclosure may be embodied in modified forms without departing from the essential characteristics of the present disclosure. Therefore, the disclosed embodiments should be considered from an illustrative point of view, not from a restrictive point of view. The scope of the present disclosure is defined by the claims rather than the foregoing description, and all differences within the scope equivalent thereto should be construed as being included in the present disclosure.

## Claims

1. A pharmaceutical composition for preventing or treating osteoarthritis, the pharmaceutical composition containing a compound represented by the following Formula 1 or a pharmaceutically acceptable salt thereof: 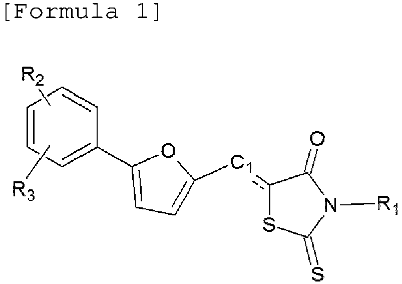
wherein R₁ is H, C₁₋₅ alkyl, C₁₋₅ alkoxy, -C₁₋₅ alkyl-carboxylic acid, -C₁₋₅ alkyl-C(=O)-C₁₋₃ alkyl, -C(=O)-C₁₋₅ alkyl, -C₁₋₂ alkyl-SO₂-C₁₋₂ alkyl, -C₁₋₂ alkyl-tetrahydrofuran, -phenyl, -C₁₋₂ alkyl-phenyl, -cyclohexyl, -dioxotetrahydrothiophene,-pyrazole, -C₁₋₃ alkyl-furan, or -CH(CO₂H)-CH₂-CO₂H, wherein the phenyl is unsubstituted or substituted with at least one substituent selected from the group consisting of C₁₋₃ alkyl, an amino group substituted with one or more C₁₋₂ alkyl groups, a halogen, -CF₃, and -NO₂;
R₂ is H, a halogen, C₁₋₃ alkyl, or -NO₂, or is linked to R₃ to form a ring;
R₃ is either any one of H, a halogen, C₁₋₃ alkyl, C₁₋₃ alkoxy, -C₁₋₅ alkyl-carboxylic acid, -C(=O)-O-R₄, -NO₂, and morpholine, or -C(=O)-NH-C(=O)-, -CH₂-O-C(=O)- or -C=C-C=C-, which is in the ortho position to R₂ and is linked to R₂ to form a ring;
R₄ is a C₁₋₄ alkyl group; and
C₁ is a sp³ hybrid carbon or a sp² hybrid carbon.

2. The pharmaceutical composition of claim 1, wherein R₃ is in the para position to the furan.

3. The pharmaceutical composition of claim 1, wherein the compound represented by Formula 1 is a compound selected from the group consisting of
1) 3-{5-[5-(4-chloro-phenyl)-furan-2-ylmethylene]-4-oxo-2-thioxo-thiazolidin-3-yl}-propionic acid,
2) 4-(5-((5-(4-(isopropoxycarbonyl)phenyl)furan-2-yl)methylene)-4-oxo-2-thioxothiazolidin-3-yl)butanoic acid,
3) 3-(5-((5-(4-chlorophenyl)furan-2-yl)methyl)-2,4-dioxothiazolidin-3-yl)propionic acid,
4) 3-(2,4-dimethylphenyl)-5-[5-(3-nitrophenyl)-furan-2-ylmethylene]-2-thioxothiazolidine-4-one,
5) 5-[5-(4-morpholine-4-yl-3-nitrophenyl)-furan-2-ylmethylene]-2-thioxothiazolidine-4-one
6) 5-[5-(4-oxo-2-thioxothiazolidine-5-ylidenemethyl)-furan-2-yl]-isoindole-1,3-dione,
7) 3-(2-methanesulfonylethyl)-5-[5-(3-nitrophenyl)-furan-2-ylmethylene]-2-thioxothiazolidine-4-one,
8) 5-[5-(4-nitrophenyl)-furan-2-ylmethylene]-3-(tetrahydrofuran-2-ylmethyl)-2-thioxothiazolidine-4-one,
9) 3-(4-diethylaminophenyl)-5-[5-(4-nitrophenyl)-furan-2-ylmethylene]-2-thioxothiazolidine-4-one,
10) 5-[5-(2,4-dichlorophenyl)-furan-2-ylmethylene]-3-(3-fluorophenyl)-2-thioxothiazolidine-4-one,
11) 3-phenethyl-5-(5-phenyl-furan-2-ylmethylene)-2-thioxothiazolidine-4-one,
12) 5-[5-(3-chloro-4-methylphenyl)-furan-2-ylmethylene]-3-ethyl-2-thioxothiazolidine-4-one,
13) 5-(naphthalen-1-yl)furan-2-ylmethylene-3-(2-oxopropyl)-2-thioxothiazolidine-4-one,
14) 3-cyclohexyl-5-[5-(2-nitrophenyl)-furan-2-ylmethylene]-2-thioxothiazolidine-4-one,
15) 3-(3-fluorophenyl)-5-[5-(4-nitrophenyl)-furan-2-ylmethylene]-2-thioxothiazolidine-4-one,
16) 3-(1,1-dioxotetrahydrothiophen-3-yl)-5-[5-(2-nitrophenyl)-furan-2-ylmethylene]-2-thioxothiazolidine-4-one,
17) 5-[5-(2-nitrophenyl)-furan-2-ylmethylene]-3-(4-oxopentyl)-2-thioxothiazolidine-4-one,
18) 5-[5-(2-bromo-5-nitrophenyl)furan-2-ylmethylene]-3-(4-iodophenyl)-2-thioxothiazolidine-4-one,
19) 5-[5-(2-chloro-4-nitrophenyl)furan-2-ylmethylene]-2-thioxo-3-(3-trifluoromethylphenyl)-thiazolidine-4-one,
20) 3-benzyl-5-[5-(4-ethoxy-2-nitrophenyl)-furan-2-ylmethylene]-2-thioxothiazolidine-4-one,
21) {5-[5-(2-methyl-5-nitrophenyl)furan-2-ylmethylene]-4-oxo-2-thioxothiazolidine-3-yl}acetic acid,
22) {5-[5-(4-ethoxy-2-nitrophenyl)-furan-2-ylmethylene]-4-oxo-2-thioxothiazolidine-3-yl}-acetic acid,
23) {5-[5-(4-methoxy-2-nitrophenyl)-furan-2-ylmethylene]-4-oxo-2-thioxothiazolidine-3-yl}acetic acid,
24) 3-(4-nitrophenyl)-5-[5-(4-nitrophenyl)-furan-2-ylmethylene]-2-thioxothiazolidine-4-one,
25) 4-[5-(4-oxo-2-thioxothiazolidine-5-ylidenemethyl)-furan-2-yl]-benzoicisopropylester,
26) 5-[5-(2,5-dichlorophenyl)furan-2-ylmethylene]-3-(1,5-dimethyl-3-oxo-2-phenyl-2,3-dihydro-1H-pyrazole-4-yl)-2-thioxothiazolidine-4-one,
27) 5-[5-(1-oxo-1,3-dihydroisobenzofuran-5-yl)-furan-2-ylmethylene]-2-thioxothiazolidine-4-one,
28) 3-(furan-2-ylmethyl)-5-[5-(2-nitrophenyl)furan-2-ylmethylene]-2-thioxothiazolidine-4-one,
29) 5-[5-(2-nitrophenyl)-furan-2-ylmethylene]-3-phenethyl-2-thioxothiazolidine-4-one,
30) 3-(3-chlorophenyl)-5-[5-(2,3-dichlorophenyl)-furan-2-ylmethylene]-2-thioxothiazolidine-4-one,
31) 2-chloro-5-[5-(4-oxo-2-thioxothiazolidine-5-ylidenemethyl)-furan-2-yl]-benzoicpropylester,
32) 4-{5-[5-(3,4-dichlorophenyl)-furan-2-ylmethylene]-4-oxo-2-thioxothiazolidine-3-yl}-butanoic acid,
33) 2-{5-[5-(4-nitrophenyl)-furan-2-ylmethylene]-4-oxo-2-thioxothiazolidine-3-yl}-succinic acid,
34) 5-[5-(4-chlorophenyl)furan-2-ylmethylene]-3-(furan-2-ylmethyl)-2-thioxothiazolidine-4-one,
35) 5-[5-(2,4-dichlorophenyl)furan-2-ylmethylene]-2-thioxo-3-(3-trifluoromethylphenyl)-thiazolidine-4-one,
36) 3-(furan-2-ylmethyl)-5-[5-(4-nitrophenyl)furan-2-ylmethylene]-2-thioxothiazolidine-4-one,
37) 3-{5-[5-(3-nitrophenyl)furan-2-ylmethylene]-4-oxo-2-thioxothiazolidine-3-yl}-propionic acid,
38) 3-{5-[5-(4-chlorophenyl)furan-2-ylmethylene]-4-oxo-2-thioxothiazolidine-3-yl}-propionic acid,
39) 4-{5-[3-(2,4-dimethylphenyl)-4-oxo-2-thioxothiazolidin-5-ylidenemethyl]-furan-2-yl}benzoic acid,
40) {5-[5-(3-nitrophenyl)furan-2-ylmethylene]-4-oxo-2-thioxothiazolidin-3-yl}acetic acid, and
41) 5-[5-(4-bromophenyl)-furan-2-ylmethylene]-3-(3-oxobutyl)-2-thioxothiazolidine-4-one.

4. The pharmaceutical composition of claim 1, wherein the osteoarthritis is osteoarthritis caused by increased expression of ZIP8.

5. The pharmaceutical composition of claim 1, wherein the compound alleviates synovitis.

6. A food composition for preventing or ameliorating osteoarthritis, the food composition containing the compound represented by Formula 1 according to claim 1.
